# EUROPÄISCHE PATENTSCHRIFT

(11) **EP 2 050 482 B1**
(45) Veröffentlichungstag und Bekanntmachung des Hinweises auf die Patenterteilung: **14.07.2010**
(21) Anmeldenummer: 08007857.9
(22) Anmeldetag: 23.04.2008
(51) Int. Cl.: A61N 2/02, A61B 17/86, H01F 5/00

(54) **Implantierbare Vorrichtung, System zum Erzeugen lokalisierter elektromagnetischer Felder im Bereich eines Implantats und Spulenanordnung**
Implantable device, system for creating localised electromagnetic fields in the area of an implant and a coil assembly
Dispositif implantable, système de production de champs électromagnétiques localisés dans la zone d'un implant et agencement de bobines

(30) Priorität: 16.10.2007 DE 102007049542
(43) Veröffentlichungstag der Anmeldung: 22.04.2009
(73) Patentinhaber: Neue Magnetodyn GmbH, 80333 München (DE)
(72) Erfinder: Kraus, Werner, 80539 München (DE); Kraus, Stephanie, 83646 Bad Tölz (DE); Stephan, Heribert, 80689 München (DE)
(74) Vertreter: Schumacher & Willsau

(56) Entgegenhaltungen:
- DE-A1- 2 636 818
- DE-C1- 19 928 449
- US-A- 3 745 995
- US-A- 4 195 367
- US-A- 4 501 265
- US-A1- 2003 050 527
- YUNOKUCHI K ET AL: "DEVELOPING A MORE FOCAL MAGNETIC STIMULATOR. ÖPART II: FABRICATING COILS AND MEASURING INDUCED CURRENT DISTRIBUTIONS" JOURNAL OF CLINICAL NEUROPHYSIOLOGY, RAVEN PRESS, NEW YORK, NY, US, Bd. 8, Nr. 1, 1. Januar 1991 (1991-01-01) , Seiten 112-120, XP000574197 ISSN: 0736-0258

## Beschreibung

Die Erfindung betrifft eine implantierbare Vorrichtung zur Befestigung an einem Knochen, ein System zum Erzeugen lokalisierter elektromagnetischer Felder im Bereich eines Implantats sowie eine in diesem Zusammenhang vorteilhaft einsetzbare Spulenanordnung.

Bei einem kompliziert gebrochenen oder pathologisch destruierten Knochen, ist die Wiederherstellung bzw. Erhaltung der ursprünglichen funktionsgerechten Form unverzichtbare Voraussetzung für seine physiologische Regeneration. In diesem Zusammenhang kommen seit mehr als sechs Jahrzehnten und noch immer in zunehmendem Maße Metallimplantate zur Anwendung, die durch Verschraubung der unverletzten Knochenareale bzw. Knochenstümpfe den Defekt überbrücken, um den Beanspruchungen durch die Muskel- und Sehnenspannung einschließlich notwendiger physiotherapeutischer Übungen standzuhalten. Diese Maßnahme der Knochenchirurgie wird als "übungsstabile Osteosynthese" bezeichnet.

Die Formerhaltung des defekten Röhrenknochens gegen den physiologisch-axialen Druck, wird mit der Implantation eines Metallrohres in der Markhöhle des langen Knochens zwischen den Gelenken erreicht, das an seinem distalen und proximalen Ende durch Querverschraubungen die Knochen-Fragmente gegen Verdrehung (Torsion) sichert. Der Hauptanwendungsbereich des Knochen-Marknagels, der nach seinem Erfinder, dem Chirurgen Gerhard Küntscher, auch Küntscher-Nagel genannt wird, sind die Läsionen im mittleren Drittel der langen Knochen (Tibia, Femur und Humerus).

Für die Stabilisierung gelenknaher, insbesondere mehrfragmentärer Läsionen ist der Knochen-Marknagel ungeeignet. In diesen Fällen wird die Wiederherstellung der Form durch die Verschraubung des Knochens und seiner Fragmente durch außen an seiner Oberfläche anliegende Stützplatten aus nichtrostendem Stahl, oder Legierungen wie Co, Cr, Mo und Titan-Legierungen versucht. Die Knochen-Stützimplantate können mit biokompatiblen Beschichtungen versehen sein, die eine hohe elektrische Leitfähigkeit aufweisen bspw. Titan-Niob-Oxinitrit. Im Unterschied zu dem durch axialen Druck belasteten Marknagel wird die quer zur langen Knochenachse verschraubte Stützplatte hauptsächlich durch Biegung beansprucht. Ihre stützende Wirkung hängt damit von ihrer Dauerschwingfestigkeit ab und wird durch diese begrenzt. Das Problem der Stützplatten-Osteosynthese stellt sich damit folgendermaßen: Bei gegebenen Material- und Formeigenschaften müssen die Stützplatte und ihre Verschraubung mit dem Knochen, den Wettlauf zwischen Knochenheilung und Implantatbruch durch die rechtzeitige belastungsstabile Konsolidierung des Knochens gewinnen, bevor die Elastizität des Stützmetalls ihr Ende erreicht hat.

Eine weitere Komplikation der Knochenheilung, die mit der Implantation von Fremdkörper-Implantaten großen Volumens verbunden ist, bildet die bakterielle Infektion. Sie entwickelt sich mit der dauerhaften Verdrängung von Blutgefäßen in der Knochenhaut (dem Periosteum) und im meist dünnen Weichgewebemantel um das Implantat. Mit der zunehmenden Resistenz bestimmter Keime (Staphylococcus aureus) gegen Antibiotika entsteht ein zentrales medizinisches Problem, das beispielsweise im Bereich der unteren Extremitäten oft nur mehr mit der Amputation zu "lösen" ist. Statistische Zahlen weisen im Bereich der distalen Tibia und des Fußes auf eine Amputationshäufigkeit von bis zu 10% hin. Dass dieser katastrophale Verlauf mit der Anwendung des Magnetodyn-Verfahrens vermieden werden kann, wurde seit über drei Jahrzehnten an abiologischen Situationen in der orthopädischen und unfallchirurgischen Klinik gezeigt. In jüngerer Zeit, besonders in den letzten drei bis vier Jahren, ist es gelungen, in grundlegenden Experimenten, in vitro, die bakterizide Wirkung und die Aktivierung der Antibiose bei resistenten Keimen durch Applikation der Elektro-Magnetfelder des Magnetodyn-Verfahrens nachzuweisen und damit die in der Klinik dokumentierten Heilerfolge bei chronisch infizierten Knochendefekten zu erklären.

Die Verwendung implantierter Knochenstützplatten und Knochenschrauben als Wechselstromelektroden durch Ihren Kontakt mit den Ausgängen einer Sekundärspule, den sogenannten Übertragern wurde bereits in der DT 1 918 299 beschrieben.

Gemäß der DT 26 11 744 A1 wurde die Technik des Magnetodyn-Verfahrens der magnetodynamisch induzierten Elektro-Osteostimulation in den Schaft und die Pfanne von Prothesen des Hüftgelenks integriert.

Der Elektro-Marknagel mit Sekundär-Spule und Elektroden im Hohlzylinder eines Knochenmarknagels wurde in der DE 26 36 818 beschrieben.

Die DE 199 28 449 Cl betrifft den Einbau der Sekundärspule in die axiale Bohrung einer Knochenschraube im elektrischen Kontakt mit dem Schaft der Schraube und einer gegen den Schaft isolierten Spitze. Dieses System wird in der Praxis als Bipolares-Induktions-Schrauben-System (BISS) bezeichnet. Die bipolare Induktionsschraube wurde als solitäres Implantat auf Schrauben-Längen von 75 bis 110 mm begrenzt. Sie wird in der chirurgischen Klinik für die magnetisch induzierte Elektro-Osteo-Stimulation beispielsweise bei Knochennekrosen des Hüftkopfes oder bei Frakturen des Oberschenkelhalses eingesetzt.

Aus der US 3,745,995 ist eine mit Schrauben an einem Knochen fixierte Platte bekannt.

Der Erfindung liegt die Aufgabe zugrunde, die Konsolidierung eines mit einem Stützimplantat ausgestatteten gebrochenen Knochens zu beschleunigen und bakterielle Probleme bei der Osteosynthese sowie der Endoprothetik zu vermeiden. Diese Aufgabe wird mit den Merkmalen der unabhängigen Ansprüche gelöst.

Vorteilhafte Ausführungsformen der Erfindung sind in den abhängigen Ansprüchen angegeben.

Die Erfindung besteht in einer implantierbaren Vorrichtung zur Befestigung an einem Knochen, mit einem mindestens zwei Durchgangsöffnungen aufweisenden elektrisch leitfähigen Basiskörper und mindestens einem Paar schaftförmiger Kontaktmittel, wobei jedes Kontaktmittel in einem implantierten Zustand eine Durchgangsöffnung des Basiskörpers durchdringt und in einen Bereich des Knochens eindringt, wobei die Oberfläche jedes Kontaktmittels einen ersten und einen zweiten elektrisch leitfähigen Oberflächenabschnitt sowie einen die elektrisch leitfähigen Oberflächenabschnitte voneinander trennenden elektrisch isolierenden Oberflächenabschnitt aufweist, wobei der erste elektrisch leitfähige Oberflächenabschnitt eines jeden Kontaktmittels in einem implantierten Zustand den Basiskörper elektrisch kontaktiert, während der zweite elektrisch leitfähige Oberflächenabschnitt eines jeden Kontaktmittels in einem implantierten Zustand gegen den Basiskörper mittels des elektrisch isolierenden Oberflächenabschnittes elektrisch isoliert ist, wobei jedes Kontaktmittel im Inneren eine Spulenanordnung enthält, mittels derer die elektrisch leitenden Oberflächenabschnitte eines jeden Kontaktmittels elektrisch gekoppelt sind, und wobei die Spulenanordnungen der Kontaktmittel eines Paars gegensinnig gewickelt sind. Wird eine solche Vorrichtung in bestimmungsgemäßer Weise an einem Knochen befestigt und einem äußeren elektromagnetischen Wechselfeld ausgesetzt, welches beispielsweise eine Magnetfeldstärke von 1 bis 5 mT und einen sinusförmigen Verlauf mit einer Frequenz von 1 bis 20 Hz aufweist, so wird in den sich entlang der schaftförmigen Kontaktmittel erstreckenden Spulenanordnungen eine elektrische Spannung induziert, wobei zu diesem Zweck das Magnetfeld eine Komponente in Richtung der Erstreckung des Kontaktmittelschaftes aufweisen muss. Da die beiden Pole einer jeweiligen Spulenanordnung mit voneinander elektrisch isolierten Bereichen der Kontaktmittel verbunden sind, wird ein elektrisches Feld zwischen den zugehörigen leitenden Oberflächenabschnitten aufgebaut. Da nun weiterhin einer der Oberflächenabschnitte mit dem elektrisch leitfähigen Basiskörper in elektrisch leitender Verbindung steht und weiterhin die Spulenanordnungen gegensinnig gewickelt sind, werden in dem Paar schaftförmiger Kontaktmittel Spannungen mit unterschiedlicher Polarität erzeugt, wobei die ersten Oberflächenabschnitte der jeweiligen Kontaktmittel auf identischem Potential liegen. Folglich hat die Spannung zwischen den jeweiligen zweiten Oberflächenabschnitten der Kontaktmittel den doppelten Betrag der in jedem einzelnem Kontaktmittel erzeugten Spannung. Da die Spannung in jedem einzelnen Kontaktmittel insbesondere durch die Induktivität der Spulenanordnung und somit auch die in Richtung des Magnetfelds vorliegende Erstreckung der Spulenanordnung begrenzt ist, gelingt es auf der Grundlage der vorliegenden Erfindung, auch mit relativ kurzbauenden Kontaktmitteln eine Spannung zu erzeugen, die sich als in physiologischer Hinsicht besonders wirksam erwiesen hat.

Die Erfindung ist in besonders nützlicher Weise dadurch weitergebildet, dass mindestens ein Kontaktmittel eines Paars eine Befestigungsschraube ist, die den Basiskörper mit einem Schraubenkopf kontaktiert und in einem implantierten Zustand mit einem Schraubenschaft in den Knochen eingeschraubt ist. Auch wenn es grundsätzlich denkbar ist, dass beliebige Befestigungsmittel oder auch andersartige schaftförmige Mittel, die nicht oder nur wenig zur Befestigung der Knochenstützplatte beitragen, mit den vorteilhaften erfindungsgemäßen Eigenschaften ausgestattet sind, ist es doch von besonderem Vorteil, die elektromagnetischen Eigenschaften der Kontaktmittel in eine Befestigungsschraube des Basiskörpers zu integrieren.

Besonders nützlich ist in diesem Zusammenhang, dass beide Kontaktmittel eines Paars Befestigungsschrauben sind.

Die Erfindung ist in besonders vorteilhafter Weise dadurch weitergebildet, dass der elektrisch isolierende Oberflächenabschnitt der Befestigungsschraube den Schraubenkopf gegen den Hauptteil des Schraubenschaftes elektrisch isoliert. Dieser Hauptteil des Schraubenschaftes ist im implantierten Zustand derjenige Teil, welcher im Knochen angeordnet ist. Folglich wird das elektrische Feld in vorteilhafter Weise über den gesamten Querschnitt des Knochens aufgebaut, wobei benachbarte Schraubenschäfte sowie die Schraubenschäfte und der Basiskörper Gegenelektroden bilden.

Von besonderem Vorteil ist es, dass mehrere Paare schaftförmiger Kontaktmittel vorgesehen sind, wobei jeweils benachbarte Kontaktmittel gegensinnig gewickelte Spulenanordnungen aufweisen. Sind die so ausgestatten Kontaktmittel beispielsweise in einer Reihe angeordnet, so kann ein elektrisches Feld über die gesamte Ausdehnung dieser Kontaktmittelreihe zur Verfügung gestellt werden.

Es kann vorgesehen sein, dass der Basiskörper ein Osteosynthesemittel ist.

In diesem Zusammenhang ist es von besonderem Vorteil, dass das Osteosynthesemittel eine implantierbare Knochenstützplatte ist. Eine solche Knochenstützplatte wird mit Schrauben am Knochen befestigt, die sich vorwiegend senkrecht zur Längsachse des Knochens erstrecken. Demzufolge haben die Schrauben eine stark begrenzte Länge, so dass auch nur eine begrenzte induzierte Spannung erzeugt werden kann. Auf der Grundlage der vorliegenden Erfindung kann diese Spannung zwischen benachbarten komplementären Schrauben verdoppelt werden. Weiterhin liegt auch eine Spannung zwischen den Schraubenschäften und der Knochenstützplatte vor, was Drucknekrosen des Knochenareals unter der Stützplatte vermeidet.

Ebenfalls kann vorgesehen sein, dass der Basiskörper eine Endoprothese ist.

In diesem Zusammenhang ist es von besonderem Vorteil, dass der Basiskörper die Pfanne eines künstlichen Gelenkes ist. Besonders die Verankerung der Pfanne eines künstlichen Hüftgelenks lässt sich so in einer Weise gestalten, dass die Vitalität und die Stabilität des Beckenknochens erhalten bleibt, indem nämlich in beliebigen Zeitintervallen die Magnetfeldinduktion auf das Implantat angewendet wird.

Die Erfindung besteht weiterhin in einem System zum Erzeugen lokalisierter elektromagnetischer Felder im Bereich eines Implantats, mit einer implantierbaren Vorrichtung zur Befestigung an einem Knochen, mit einem mindestens zwei Durchgangsöffnungen aufweisenden elektrisch leitfähigen Basiskörper und mindestens einem Paar schaftförmiger Kontaktmittel, wobei jedes Kontaktmittel in einem implantierten Zustand eine Durchgangsöffnung des Basiskörpers durchdringt und in einen Bereich des Knochens eindringt, wobei die Oberfläche jedes Kontaktmittels einen ersten und einen zweiten elektrisch leitfähigen Oberflächenabschnitt sowie einen die elektrisch leitfähigen Oberflächenabschnitte voneinander trennenden elektrisch isolierenden Oberflächenabschnitt aufweist, wobei der erste elektrisch leitfähige Oberflächenabschnitt eines jeden Kontaktmittels in einem implantierten Zustand den Basiskörper elektrisch kontaktiert, während der zweite elektrisch leitfähige Oberflächenabschnitt eines jeden Kontaktmittels in einem implantierten Zustand gegen den Basiskörper mittels des elektrisch isolierenden Oberflächenabschnitt elektrisch isoliert ist, wobei jedes Kontaktmittel im Inneren eine Sekundärspulenanordnung enthält, mittels derer die elektrisch leitenden Oberflächenabschnitte eines jeden Kontaktmittels elektrisch gekoppelt sind, und wobei die Sekundärspulenanordnungen der Kontaktmittel eines Paars gegensinnig gewickelt sind, und mit einer Primärspulenanordnung, die ein elektromagnetisches Feld im Bereich der Kontaktmittel erzeugt, sowie einem Schwingungsgenerator zum Erzeugen eines Wechselstroms in der Primärspulenanordnung. Die kortikale Fixierung durch Verschraubung des lädierten Knochens mit einer Stützplatte bestimmt die Richtung der Schrauben im Wesentlichen senkrecht zur langen Knochenachse. Dieser muss zwangsläufig auch die Richtung des äußeren induzierenden ElektroMagnetfeldes folgen. Statt der beispielsweise für die Induktion des Elektronagels oder der langen bipolaren Schrauben benötigten Solenoid-Spulen, die den Läsionsbereich des Knochens umhüllen und damit die Richtung des induzierenden Elektromagnetfeldes parallel zur langen Körper- und Knochenachse vorgeben, sind für die Induktion der Stützplatten-Schrauben zwei Magnetspulen in Helmholtzanordnung beiderseits des lädierten Knochens notwendig.

In diesem Zusammenhang ist es besonders nützlich, dass die Primärspulenanordnung eine Spulenwicklung mit einem Kreuzungspunkt aufweist, die durch eine achtförmige Gestalt zwei Flächen definiert, wobei die Flächen zumindest während der Anwendung der Primärspulenanordnung so zueinander ausgerichtet sind, dass die von einem durch einen Stromfluss in der Primärspulenanordnung erzeugten, die Flächen durchdringenden Magnetfelder im Wesentlichen gleichgerichtet sind. Es lässt sich also mit einer einzigen Spule ein Magnetfeld erzeugen, das im Hinblick auf senkrecht zur Knochenachse angeordnete Schrauben eine geeignete Ausrichtung hat. Die Wirkung von zwei getrennten Induktionsspulen mit gleicher Richtung ihres Magnetfeldes, in dem die lädierte Körperregion gelagert wird, kann auf diese Weise auch mit einer einzigen Spule erreicht werden.

Nützlicherweise ist vorgesehen dass die Primärspulenanordnung elastisch verformbar ist, so dass die Flächen auf gegenüberliegenden Seiten der implantierbaren Vorrichtung angeordnet werden können. Durch ihre elastische Wicklung wird die Umformung im Sinne eines Achter- oder Unendlich-Zeichens ermöglicht. Die sich durch diese Umformung der Spulenwicklung ergebenden "schlaufen" der Spule können an beiden Seiten einer Extremität angelegt werden.

Beispielsweise kann vorgesehen sein, dass an zwei dem Kreuzungspunkt abgewandten Positionen der Primärspulenanordnung zusammenwirkende Befestigungsmittel vorgesehen sind, durch die die Ausrichtung der Flächen zueinander geschaffen und aufrechterhalten werden kann. Die Befestigungsmittel können beispielsweise mittels Gurten, Druckknöpfen, Klettverschlüssen, Schnallen und dergleichen realisiert sein.

In besonders vorteilhafter Weise kann vorgesehen sein, dass der Kreuzungspunkt der Primärspulenanordnung durch eine Kopplungseinrichtung fixierbar ist. Eine solche Kopplungseinrichtung kann beispielsweise durch ein elastisches Band mit Klettverschluss oder Gürtelschließe realisiert sein.

Es ist von besonderem Vorteil, dass die Lage des Kreuzungspunktes und somit die Maße der Flächen variabel sind. Durch das auf diese Weise wählbare Flächenverhältnis der Spulenschlaufen ist die magnetische Induktionsflussdichte, die als Quotient aus dem magnetischen Fluss und der betrachteten Fläche definiert ist, einstellbar. Das Verhältnis der Induktionsflussdichten ist der Kehrwert des Verhältnisses der jeweils zugehörigen Flächen. Das Flächenverhältnis der beiden Schlaufen kann nach den therapeutischen Erfordernissen gewählt werden, indem eine veränderbare Fixierung des Kreuzungspunktes durch variable Anordnung der Kopplungseinrichtung ermöglicht wird. Beispielsweise kann in einem Zielbereich des Körpers eine hohe magnetische Induktionsflussdichte dadurch erzielt werden, dass eine kleinflächige Spulenschlaufe in dessen Nähe gebracht wird, während im Hinblick auf die andere großflächige Spulenfläche zur Bereitstellung des Helmholtzspuleneffektes hauptsächlich auf deren parallele Anordnung zur kleineren Fläche zu achten ist.

Die Erfindung bezieht sich weiterhin auf eine Spulenanordnung, insbesondere zur Verwendung in einem erfindungsgemäßen System zum Erzeugen lokalisierter elektromagnetischer Felder im Bereich eines Implantats, mit einer einen Kreuzungspunkt aufweisenden Spulenwicklung, die durch eine achtförmige Gestalt zwei Flächen definiert, wobei die Flächen so zueinander ausgerichtet sind, dass die von einem durch einen Stromfluss in der Spulenanordnung erzeugten, die Flächen durchdringenden Magnetfelder im Wesentlichen gleichgerichtet sind. Auf diese Weise werden die Vorteile und Besonderheiten des erfindungsgemäßen Systems auch im Rahmen einer Spulenanordnung realisiert. Dies gilt auch für die nachfolgend angegebenen besonders bevorzugten Ausführungsformen der erfindungsgemäßen Spulenanordnung.

Diese ist in nützlicher Weise dadurch weitergebildet, dass die Spulenanordnung elastisch verformbar ist, so dass die Flächen auf gegenüberliegenden Seiten einer implantierbaren Vorrichtung angeordnet werden können.

Weiterhin ist vorgesehen, dass an zwei dem Kreuzungspunkt abgewandten Positionen der Spulenanordnung zusammenwirkende Befestigungsmittel vorgesehen sind, durch die die Ausrichtung der Flächen zueinander geschaffen und aufrechterhalten werden kann.

In besonders vorteilhafter weise kann die Spulenanordnung so weitergebildet sein, dass der Kreuzungspunkt der Spulenanordnung durch eine Kopplungseinrichtung fixierbar ist.

Es ist weiterhin von besonderem Vorteil, dass die Lage des Kreuzungspunktes und somit die Maße der Flächen variabel sind.

Die Erfindung berücksichtigt somit neue Erkenntnisse der biomechanischen Gegebenheiten der Stützplatten-Schrauben-Osteosynthese und ihrer kausalen Verknüpfung mit der chronischen Infektion. Sie dient beispielsweise der Stabilisierung gelenknaher Mehrfachfrakturen langer Röhrenknochen, für die der Elektronagel keine ausreichende Stabilität bietet. Die Geometrie der Schrauben, der Sekundärspulen und der Elektroden wird gegenüber den Vorrichtungen des Standes der Technik grundlegend verändert, um in Verbindung mit der Stützplatte eine therapeutisch wirksame Verteilung der elektrischen Felder und Ströme innerhalb großer Trümmerzonen eines Knochens erzeugen zu können. Das Gleiche gilt für die Dimensionierung und die Verteilung der stimulierenden elektrischen Felder im Bereich der Pfanne eines künstlichen Hüftgelenkes im Beckenknochen. Die erfindungsgemäßen Bipolaren Induktionsschrauben besitzen eine Länge von beispielsweise 30 bis 50 mm, vorzugsweise von 40 mm und einen Durchmesser von beispielsweise 4 bis 6 mm. Die Isolierstrecke der kurzen Bipolarschrauben befindet sich, anders als bei den langen Bipolarschrauben (siehe z. B. DE 199 28 449 C1), zwischen dem Kopf und dem Schaft der Schraube. Die Wicklung um den Fe-Kern der Sekundärspule ist in der Weise angeordnet, dass der Wickelsinn der Sekundärinduktivität von jeweils zwei Schrauben entgegengesetzt, d.h. komplementär, gerichtet ist. Bei der Verschraubung einer Stützplatte mit dem Knochen werden die Köpfe der Bipolarschrauben miteinander elektrisch verbunden, und damit die Induktionsspulen mit jeweils gegenläufiger oder komplementärer Wicklung hintereinander geschaltet. In einem anwendbaren Elektro-Magnetfeld mit einer Feldstärke von beispielsweise 1 bis 5 mT und einer sinusförmigen Erregerfrequenz von 1 bis 20 Hz wird mit dieser Anordnung die induzierte elektrische Spannung zwischen den Schäften von zwei komplementären Schrauben verdoppelt. Zwischen dem Schaft und der Stützplatte im Kontakt mit dem Schraubenkopf, entsteht jeweils nur die einfache elektrische Spannung, die in einer Bipolarschraube induziert wird.

Die Vorteile, die bei der Osteosynthese und der Gelenkendoprothetik durch Verschraubung der Metallstützplatten bzw. der Pfanne eines künstlichen Hüftgelenks mit mindestens zwei oder mehr der erfindungsgemäßen Biopolarschrauben erreicht werden können, sind insbesondere:
- Die Induktion gleich hoher Spannungen von 500 mV bis 700 mV im Knochen, die sich bei der solitären Anwendung der 75 mm bis 110 mm langen Biopolarschrauben optimal bewährt haben, wird durch die erfindungsgemäße Anwendung von mindestens zwei der 30 mm bis 50mm langen, also kürzeren Biopolarschrauben mit komplementärer Sekundärinduktivität bei der Fixierung einer Stützplatte am Knochen erreicht.
- Die Anordnung mehrerer komplementärer Biopolarschrauben in wechselnder Reihenfolge ermöglicht die Ausdehnung des induzierten elektrischen Feldes über die ganze Länge einer Stützplatte zur Stabilisierung und Therapie großer Defekte.
- Die Verteilung des elektrischen Feldes erfolgt über den ganzen Querschnitt des Knochens.
- Es kommt zu einer Induktion einer elektrischen Spannung von ca. 250 mV bis 350 mV zwischen dem Schaft der einzelnen Biopolarschraube und der Knochenstützplatte. Damit können Drucknekrosen des Knochenareals unter der Stützplatte vermieden werden.
- Bei der Verankerung der Pfanne eines künstlichen Hüftgelenkes ist besonders die Erhaltung der Vitalität und Stabilität des Beckenknochens durch die Wiederholung der Magnetfeld-Induktion der bipolaren Pfannenverschraubung in beliebigen Zeitintervallen möglich.
- Unabhängig von der konkreten Anwendung kommt es zu einer drastischen Verringerung der Infektionsgefahr bei allen Kurz- und Langzeitimplantaten der Osteosynthese und der Gelenkendoprothetik.

Mit der erfindungsgemäßen Spulenanordnung wird zusätzlich erreicht, dass das Magnetfeld der beiden sich gegenüberstehenden Schlaufen der Spulenanordnung durch die räumliche Umkehr der Stromrichtung in einer der beiden Schlaufen, wie bei der Anordnung von zwei getrennten Spulen nach Helmholtz, gleichgerichtet ist. Eine solche Spule, die zusätzlich elastisch formbar ist, ermöglicht die Induktion implantierter Sekundärspulen weitgehend unabhängig von ihrer Ausrichtung zur langen Knochenachse. Eine einfache Handhabung bei der Anpassung der geometrischen Form der Spulenanordnung an die Lage der jeweiligen Knochen bzw. Weichgewebeläsion wird ermöglicht. Beispielsweise können die Schlaufenflächen an Behandlungsbereiche wie Fuß, Knie Unterschenkel, Oberschenkel, Becken, Wirbelsäule, Hand, Unterarm, Oberarm, Kiefer und Schädelbereich angepasst werden; durch Variation der Schlaufengrößen auch im Hinblick auf die Stärke des Magnetfeldes. Eine weitere Eigenschaft der erfindungsgemäßen Spulenanordnung ist eine Zunahme der Flussdichte im Nahbereich des Kreuzungspunktes, so dass eine relativ starke Magnetfeldkonzentration auf eine kleine Körperfläche ermöglicht wird. Auf diese Weise lassen sich stark lokalisierte Erkrankungen, wie zum Beispiel Abszesse und Infekte, wirkungsvoll behandeln.

Die Erfindung wird nun mit Bezug auf die begleitenden Zeichnungen anhand besonders bevorzugter Ausführungsformen beispielhaft erläutert.

Es zeigen:
- Figur 1: eine perspektivische Darstellung eins frakturier- ten Knochens mit einer durch Knochenschrauben fi- xierten Knochenstützplatte;
- Figur 2: eine Schnittdarstellung einer erfindungsgemäßen implantierbaren Vorrichtung;
- Figur 3: eine Schnittdarstellung einer erfindungsgemäßen implantierbaren Vorrichtung, nämlich einer Kno- chenstützplatte, im implantierten Zustand mit von außen angelegtem Magnetfeld;
- Figur 4: eine perspektivische Darstellung einer erfin- dungsgemäßen implantierbaren Vorrichtung, nämlich einer Pfanne einer Hüftgelenkprothese;
- Figur 5: eine elastische Spulenanordnung;
- Figur 6: eine erfindungsgemäße elastische Spulenanordnung in einem Zustand außerhalb ihrer Anwendung;
- Figur 7: eine perspektivische Darstellung einer erfin- dungsgemäßen Spulenanordnung während ihrer Anwen- dung als Primärspulenanordnung; und
- Figur 8: eine schematische Darstellung zur Erläuterung der räumlichen Ausrichtung eines durch eine erfin- dungsgemäße Spulenanordnung erzeugten Magnetfel- des.

Bei der nachfolgenden Beschreibung der Zeichnungen bezeichnen gleiche Bezugszeichen gleiche oder vergleichbare Komponenten.

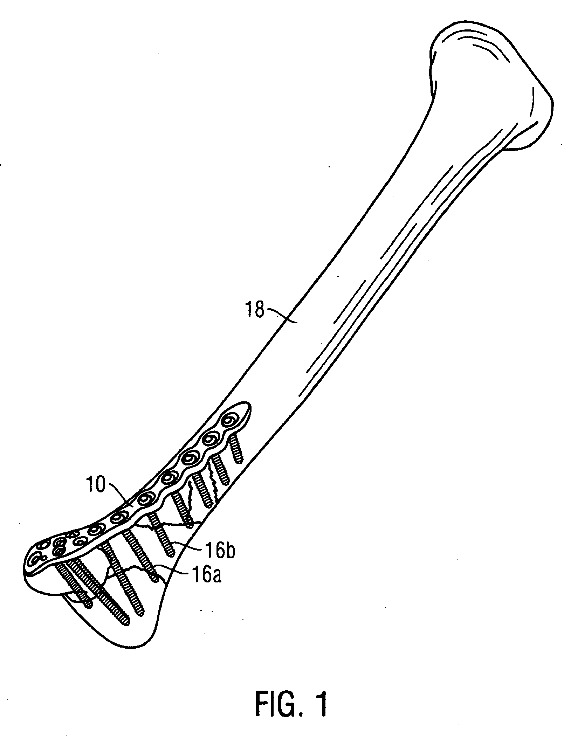

Figur 1 zeigt eine perspektivische Darstellung eins frakturierten Knochens mit einer durch Knochenschrauben fixierten Knochenstützplatte. Der Knochen 18 ist im Bereich der Frakturen mit einer Knochenstützplatte 10 stabilisiert. Diese Knochenstützplatte 10 ist mit Befestigungsschrauben an dem Knochen 18 befestigt, wobei zumindest einige dieser Befestigungsschrauben, beispielsweise das Schraubenpaar 16a, 16b ein paar schaftförmiger Kontaktmittel mit darin liegenden gegensinnig gewickelten Spulenanordnungen darstellt, wobei ein solches Schraubenpaar auch als Komplementär-Bipolarschraubenpaar bezeichnet werden kann. Grundsätzlich können alle verwendeten Schrauben in das Konzept der Komplementär-Bipolarschraubenpaare einbezogen werden. Ebenfalls ist es denkbar, dass dieses nur bei einigen oder nur bei zwei dieser Schrauben realisiert ist. Ebenfalls ist möglich, andere Mittel als Schrauben in das Konzept der komplementären Kontaktmittel einzubeziehen.

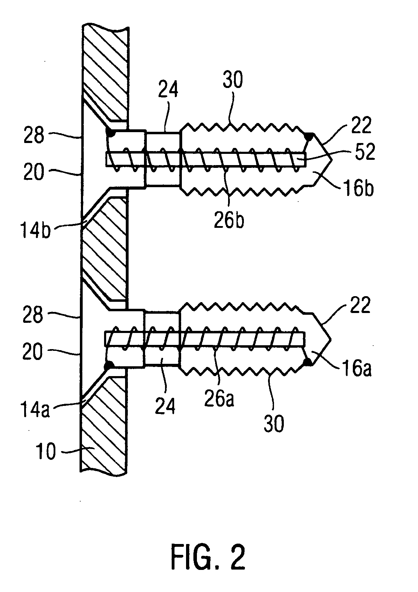

Figur 2 zeigt eine Schnittdarstellung einer erfindungsgemäßen implantierbaren Vorrichtung. Es ist ein Basiskörper 10, beispielsweise eine Knochenstützplatte, zu erkennen. Dieser Basiskörper 10 hat zwei Durchgangsöffnungen 14a, 14b, die von jeweils einer Knochenschraube 16a, 16b durchdrungen werden. Der Kopf 28 einer jeweiligen Knochenschraube 16a, 16b kontaktiert die Knochenstützplatte 10, während der Schraubenschaft 30 der Knochenschrauben 16a, 16b im Knochen angeordnet wird und über einen elektrisch isolierenden Oberflächenabschnitt 24 gegen den Schraubenkopf 28 und somit auch gegen die Knochenstützplatte 10 isoliert ist. Auf diese Weise liegen zwei voneinander isolierte elektrisch leitende Oberflächenabschnitte 20, 22 vor, welche unter Einbeziehung der Knochenstützplatte 10 Gegenelektroden zur Erzeugung eines elektrischen Feldes bilden. Innerhalb jeder Knochenschraube 16a, 16b ist eine Spulenanordnung 26a, 26b vorgesehen, die vorzugsweise auf einen Ferritkern 52 aufgewickelt ist. Die Pole der Spulenanordnung 26a, 26b kontaktieren die gegeneinander elektrisch isolierten Bereiche der Knochenschrauben 16a, 16b, so dass im Falle der Anwesenheit eines die Spulenwicklungen durchsetzenden Magnetfeldes die gegeneinander isolierten Abschnitte der Knochenschrauben 16a, 16b auf unterschiedliches Potential gebracht werden. Da die Schraubenköpfe 28 beide mit der Knochenstützplatte 10 in Verbindung stehen, befinden sich diese auf demselben elektrischen Potential. Da weiterhin die Spulenanordnungen 26a, 26b gegensinnig gewickelt sind, sind die Potentiale der Schraubenschäfte 30 bezüglich des Potentials der Schraubenköpfe entgegengesetzt. Wird in beiden Knochenschrauben 16a, 16b die betragsmäßig gleiche Spannung induziert, so beträgt die Potentialdifferenz zwischen den Schraubenschäften 30 das Doppelte der in den jeweiligen Schrauben 16a, 16b induzierten Einzelspannungen.

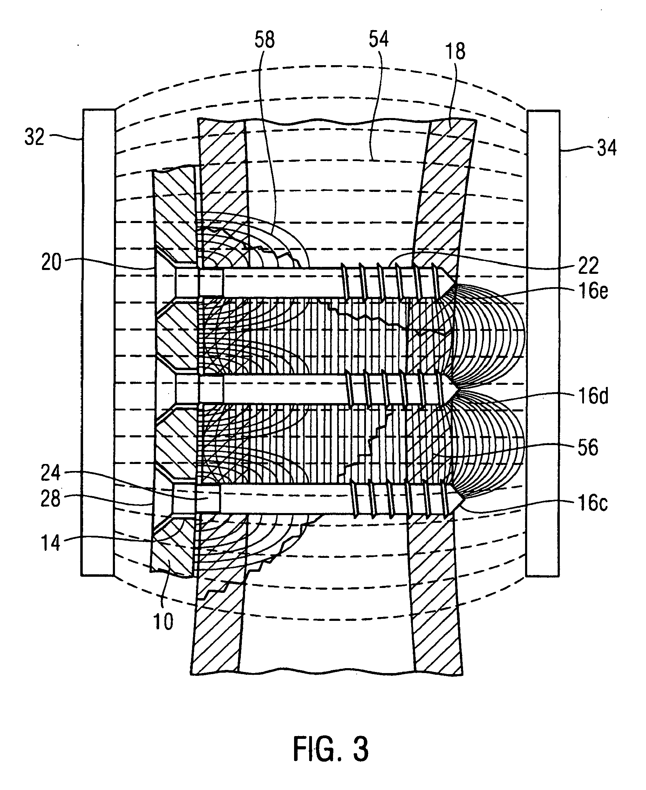

Figur 3 zeigt eine Schnittdarstellung einer erfindungsgemäßen implantierbaren Vorrichtung, nämlich einer Knochenstützplatte, im implantierten Zustand mit von außen angelegtem Magnetfeld. An dem Knochen 18 ist eine Knochenstützplatte 10 durch drei Knochenschrauben 16c, 16d, 16e befestigt. Die Knochenschrauben 16c, 16d, 16e sind gleich oder ähnlich aufgebaut, weshalb die weitere Beschreibung exemplarisch anhand einer der Knochenschrauben 16c erfolgt. Die Knochenschraube 16c verläuft durch eine Durchgangsöffnung 14 der Knochenstützplatte 10. Benachbarte Knochenschrauben enthalten jeweils gegensinnig gewickelte Spulenanordnungen und sind ansonsten vergleichbar zu den im Zusammenhang mit Figur 2 beschriebenen Knochenschrauben aufgebaut. Außerhalb der Anordnung sind zwei Magnetspulen 32, 34 angeordnet, wobei diese nach Art eines Helmholtzspulenpaars gewickelt sind, so dass die durch unterbrochene Linien dargestellten Magnetfeldlinien 54 im Wesentlichen parallel zu der Längserstreckung der Knochenschrauben 16c verlaufen. Das durch die Spulen 32, 34 erzeugte elektromagnetische Wechselfeld generiert eine elektrische Spannung in jeder der Knochenschrauben 16c, 16d, 16e wobei die dann vorliegenden elektrischen Potentiale aufgrund der gemeinsamen Kontaktierung der Schraubenköpfe 28 durch die Knochenstützplatte 10 definiert sind. Die den Heilungsprozess begünstigenden elektrischen Felder 56, 58, die mittels durchgezogener Linien gezeigt sind, bilden sich sowohl zwischen den Schraubenschäften 30 der jeweiligen Knochenschrauben 16c, 16d, 16e aus als auch zwischen den Schäften 30 jeder Knochenschraube 16c, 16d, 16e und der damit befestigten Knochenstützplatte 10. Die elektrischen Feldlinien 56 zwischen den Schraubenschäften 30 der einzelnen Schrauben 16c, 16d, 16e haben geringere Abstände als die elektrischen Feldlinien zwischen den Schraubenschäften 30 und der Knochenstützplatte 10. Dies deutet an, dass bei typischen Abständen der Komponenten sowie typischer Komponentengestalt die durch die Differenz zwischen den Schraubenpotentialen erzeugten elektrischen Felder größer sind als die durch die geringere Potentialdifferenz zwischen den Schraubenschäften 30 und der Knochenstützplatte 10 erzeugten elektrischen Felder. Tatsächlich hat das durch die verschiedenen beteiligten Potentiale erzeugte elektrische Feld eine einheitliche Gestalt, so dass beispielsweise durch das Potential der Knochenstützplatte 10 eine Verzerrung des durch die Schraubenschäfte 30 erzeugten elektrischen Feldes erfolgt; die sich in diesem Bereich überkreuzend dargestellten Feldlinien 56,58 veranschaulichen nur die dortige Anwesenheit eines komplexen Feldverlaufs. Weiterhin ist zu erwähnen, dass der Beitrag der Potentialdifferenz zwischen den Schraubenschäften 30 und der Knochenstützplatte 10 zum elektrischen Feld mit sich vergrößerndem Abstand von der Knochenstützplatte 10 abnimmt, obwohl dies die Darstellung der Feldliniendichte der Einfachheit halber nicht widerspiegelt.

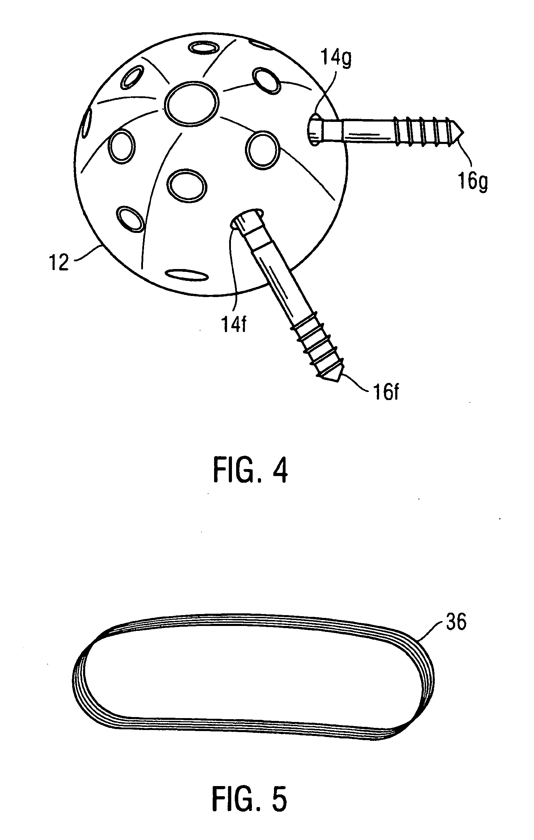

Figur 4 zeigt eine perspektivische Darstellung einer erfindungsgemäßen implantierbaren Vorrichtung, nämlich einer Pfanne einer Hüftgelenkprothese. Die Pfanne 12 des künstlichen Hüftgelenks hat eine Vielzahl von Durchgangsöffnungen, wobei durch die Durchgangsöffnungen 14f, 14g Knochenschrauben 16f, 16g hindurchgeführt sind, die im Wesentlichen vergleichbar zu den Knochenschrauben gemäß Figur 2 aufgebaut sind. Folglich können die mit Bezug auf die Figuren 1 und 3 und im Zusammenhang mit der Osteosynthese beschriebenen Vorzüge der vorliegenden Erfindung auch im Zusammenhang mit Endoprothesen zur Wirkung kommen.

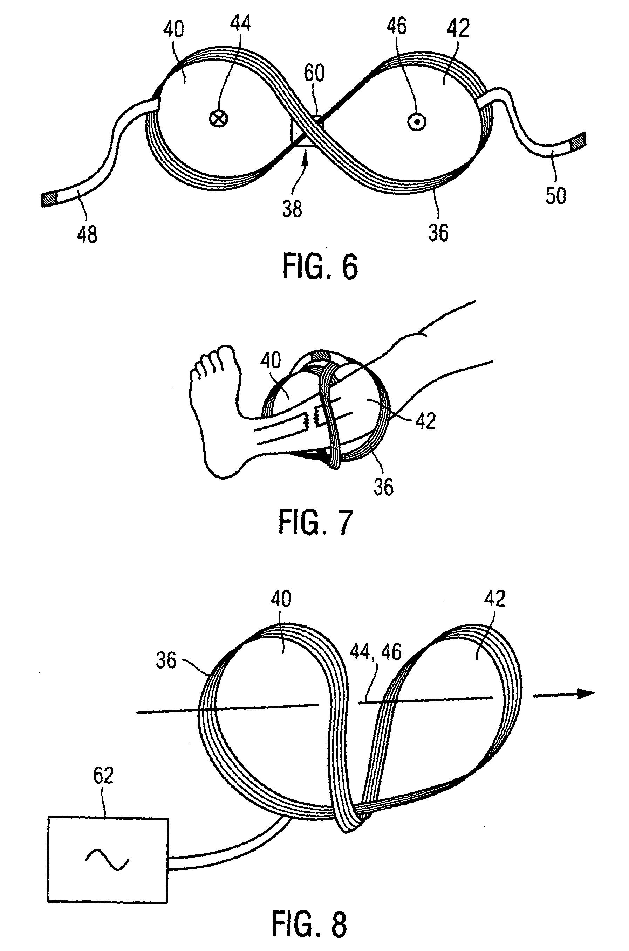

Figur 5 zeigt eine elastische Spulenanordnung. Figur 6 zeigt eine erfindungsgemäße elastische Spulenanordnung in einem Zustand außerhalb ihrer Anwendung. Figur 7 zeigt eine perspektivische Darstellung einer erfindungsgemäßen Spulenanordnung während ihrer Anwendung als Primärspulenanordnung. Figur 8 zeigt eine schematische Darstellung zur Erläuterung der räumlichen Ausrichtung eines durch eine erfindungsgemäße Spulenanordnung erzeugten Magnetfeldes. Nutzt man die Elastizität der Spulenanordnung 36 dazu aus, diese in eine achtförmige Gestalt zu bringen, so werden zwei Flächen 40, 42 sowie ein Kreuzungspunkt 38 definiert. Der Kreuzungspunkt 38 ist durch eine Kopplungseinrichtung 60 fixiert und kann vorzugsweise verschoben werden, so dass das Verhältnis der Flächen 40, 42 variabel ist. Beispielsweise kann als Kopplungseinrichtung 60 ein elastisches Band mit Klettverschluss oder Gürtelschließe dienen. Ebenfalls ist es denkbar, Oberflächen der Spulenanordnung so auszustatten, dass sie die Komponenten eines Klettverschlusses bilden und auf diese Weise an verschiedenen Stellen direkt aufeinander Haften können. Um die Einstellung zu erleichtern, können im Variationsbereich Vermerke oder Skalen vorgesehen sein; an diesen kann sich der Anwender orientieren, wenn er eine bestimmte Fixierung des Kreuzungspunktes 38 vornehmen möchte. Fließt durch eine solche Spulenanordnung 36 ein Wechselstrom, so haben die induzierten Magnetfelder entgegengesetzte Richtungen, wie durch die Vektorsymbole 44, 46 angedeutet ist. Aufgrund der Elastizität der Spulenanordnung 36 kann diese aber auch in eine andere Gestalt gebracht werden. Dies ist im Zusammenhang mit Figur 7 dargestellt, wo auch ein Bein mit einem frakturierten Knochen gezeigt ist. Nun liegen sich die Flächen 40 ,42 gegenüber und die in den jeweiligen Segmenten der Spule erzeugten Magnetfelder haben die gleiche Richtung. Dies ist in Figur 8, in der auch ein mit der Spulenanordnung gekoppelter Wechselstromgenerator 62 gezeigt ist, nochmals veranschaulicht, nämlich durch den einheitlichen Magnetfeldvektor 44, 46, der beide Flächen 40, 42 durchdringt.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste:

- 10: Basiskörper / Knochenstützplatte
- 12: Basiskörper / Endoprothese / Pfanne
- 14: Durchgangsöffnung
- 16: Kontaktmittel / Befestigungsmittel
- 18: Knochen
- 20: Oberflächenabschnitt
- 22: Oberflächenabschnitt
- 24: Oberflächenabschnitt / Isolierung
- 26: Spulenanordnung / Sekundärspulenanordnung
- 28: Schraubenkopf
- 30: Schraubenschaft
- 32: Primärspulenanordnung / Magnetspule
- 34: Primärspulenanordnung / Magnetspule
- 36: Primärspulenanordnung
- 38: Kreuzungspunkt
- 40: Fläche
- 42: Fläche
- 44: Magnetfeld / Magnetfeldvektor / Vektorsymbol
- 46: Magnetfeld / Magnetfeldvektor / Vektorsymbol
- 48: Befestigungsmittel
- 50: Befestigungsmittel
- 52: Ferritkern
- 54: Magnetfeld
- 56: elektrisches Feld
- 58: elektrisches Feld
- 60: Kopplungseinrichtung
- 62: Wechselstromgenerator

## Patentansprüche

1. Implantierbare Vorrichtung zur Befestigung an einem Knochen, mit
- einem mindestens zwei Durchgangsöffnungen (14) aufweisenden elektrisch leitfähigen Basiskörper (10, 12) und
- mindestens einem Paar schaftförmiger Kontaktmittel (16), wobei jedes Kontaktmittel (16) in einem implantierten Zustand eine Durchgangsöffnung (14) des Basiskörpers (10, 12) durchdringt und in einen Bereich des Knochens (18) eindringt,
- wobei die Oberfläche jedes Kontaktmittels (16) einen ersten und einen zweiten elektrisch leitfähigen Oberflächenabschnitt (20, 22) sowie einen die elektrisch leitfähigen Oberflächenabschnitte (20, 22) voneinander trennenden elektrisch isolierenden Oberflächenabschnitt (24) aufweist,
- wobei der erste elektrisch leitfähige Oberflächenabschnitt (20) eines jeden Kontaktmittels (16) in einem implantierten Zustand den Basiskörper (10, 12) elektrisch kontaktiert, während der zweite elektrisch leitfähige Oberflächenabschnitt (22) eines jeden Kontaktmittels (16) in einem implantierten Zustand gegen den Basiskörper (10, 12) mittels des elektrisch isolierenden Oberflächenabschnittes (24) elektrisch isoliert ist,
- wobei jedes Kontaktmittel (16) im Inneren eine Spulenanordnung (26) enthält, mittels derer die elektrisch leitenden Oberflächenabschnitte (20, 22) eines jeden Kontaktmittels elektrisch gekoppelt sind, und
- wobei die Spulenanordnungen (26) der Kontaktmittel (16) eines Paars gegensinnig gewickelt sind.

2. Implantierbare Vorrichtung nach Anspruch 1, **dadurch gekennzeichnet, dass** mindestens ein Kontaktmittel (16) eines Paars eine Befestigungsschraube ist, die den Basiskörper (10) mit einem Schraubenkopf (28) kontaktiert und mit einem Schraubenschaft (30) in den Knochen (18) einschraubbar ist.

3. Implantierbare Vorrichtung nach Anspruch 1 oder 2, **dadurch gekennzeichnet, dass** beide Kontaktmittel (16) eines Paars Befestigungsschrauben sind.

4. Implantierbare Vorrichtung nach Anspruch 2 oder 3, **dadurch gekennzeichnet, dass** der elektrisch isolierende Oberflächenabschnitt der Befestigungsschraube (16) den Schraubenkopf (28) gegen den Hauptteil des Schraubenschaftes (30) elektrisch isoliert.

5. Implantierbare Vorrichtung nach einem der vorangehenden Ansprüche, **dadurch gekennzeichnet, dass** mehrere Paare schaftförmiger Kontaktmittel (16) vorgesehen sind, wobei jeweils benachbarte Kontaktmittel (16) gegensinnig gewickelte Spulenanordnungen (26) aufweisen.

6. Implantierbare Vorrichtung nach einem der vorangehenden Ansprüche, **dadurch gekennzeichnet, dass** der Basiskörper ein Osteosynthesemittel (10) ist.

7. Implantierbare Vorrichtung nach Anspruch 6, **dadurch gekennzeichnet, dass** das Osteosynthesemittel eine implantierbare Knochenstützplatte (10) ist.

8. Implantierbare Vorrichtung nach einem der Ansprüche 1 bis 5, **dadurch gekennzeichnet, dass** der Basiskörper eine Endoprothese (12) ist.

9. Implantierbare Vorrichtung nach Anspruch 8, **dadurch gekennzeichnet, dass** der Basiskörper die Pfanne (12) eines künstlichen Gelenkes ist.

10. System zum Erzeugen lokalisierter elektromagnetischer Felder im Bereich eines Implantats, mit einer
- implantierbaren Vorrichtung zur Befestigung an einem Knochen, insbesondere nach einem der vorhergehenden Ansprüche, mit
- einem mindestens zwei Durchgangsöffnungen (14) aufweisenden elektrisch leitfähigen Basiskörper (10, 12) und
- mindestens einem Paar schaftförmiger Kontaktmittel (16), wobei jedes Kontaktmittel (16) in einem implantierten Zustand eine Durchgangsöffnung (14) des Basiskörpers (10, 12) durchdringt und in einen Bereich des Knochens (18) eindringt,
- wobei die Oberfläche jedes Kontaktmittels (16) einen ersten und einen zweiten elektrisch leitfähigen Oberflächenabschnitt (20, 22) sowie einen die elektrisch leitfähigen Oberflächenabschnitte (20, 22) voneinander trennenden elektrisch isolierenden Oberflächenabschnitt (24) aufweist,
- wobei der erste elektrisch leitfähige Oberflächenabschnitt (20) eines jeden Kontaktmittels (16) in einem implantierten Zustand den Basiskörper (10, 12) elektrisch kontaktiert, während der zweite elektrisch leitfähige Oberflächenabschnitt (22) eines jeden Kontaktmittels (16) in einem implantierten Zustand gegen den Basiskörper (10, 12) mittels des elektrisch isolierenden Oberflächenabschnitt (24) elektrisch isoliert ist,
- wobei jedes Kontaktmittel (16) im Inneren eine Sekundärspulenanordnung (26) enthält, mittels derer die elektrisch leitenden Oberflächenabschnitte (20, 22) eines jeden Kontaktmittels elektrisch gekoppelt sind, und
- wobei die Sekundärspulenanordnungen (26) der Kontaktmittel (16) eines Paars gegensinnig gewickelt sind, und mit
- einer Primärspulenanordnung (32, 34, 36), die eingerichtet ist, ein elektromagnetisches Feld im Bereich der Kontaktmittel (16) zu erzeugen, sowie
- einem Schwingungsgenerator (62) zum Erzeugen eines Wechselstroms in der Primärspulenanordnung (32, 34, 36).

11. System nach Anspruch 10, **dadurch gekennzeichnet, dass** die Primärspulenanordnung (36) eine Spulenwicklung mit einem Kreuzungspunkt (38) aufweist, die durch eine achtförmige Gestalt zwei Flächen (40, 42) definiert, wobei die Flächen (40, 42) zumindest während der Anwendung der Primärspulenanordnung (36) so zueinander ausgerichtet sind, dass die von einem durch einen Stromfluss in der Primärspulenanordnung (36) erzeugten, die Flächen durchdringenden Magnetfelder (44, 46) im Wesentlichen gleichgerichtet sind.

12. System nach Anspruch 11, **dadurch gekennzeichnet, dass** die Primärspulenanordnung (36) elastisch verformbar ist, so dass die Flächen auf gegenüberliegenden Seiten der implantierbaren Vorrichtung anordenbar sind:

13. System nach Anspruch 11 oder 12, **dadurch gekennzeichnet, dass** an zwei dem Kreuzungspunkt (38) abgewandten Positionen der Primärspulenanordnung zusammenwirkende Befestigungsmittel (48, 50) vorgesehen sind, durch die die Ausrichtung der Flächen (40, 42) zueinander schaff- und aufrechterhaltbar ist.

14. System nach Anspruch 12 oder 13, **gekennzeichnet durch** eine Kopplungsreinrichtung (60), **durch** welche der Kreuzungspunkt (38) der Primärspulenanordnung fixierbar ist.

15. System nach einem der Ansprüche 12 bis 14, **dadurch gekennzeichnet, dass** die Lage des Kreuzungspunktes (38) und somit die Maße der Flächen (40, 42) variabel sind.

## Claims

1. An implantable device to be fixed to a bone and comprising
- an electrically conductive base body (10, 12) provided with at least two through holes (14) and
- at least one pair of shaft-shaped contact means (16), wherein each contact means (16) passes through a through hole (14) of the base body (10, 12) and penetrates a section of the bone (18) in an implanted state,
- wherein the surface of each contact means (16) comprises a first and a second electrically conductive surface section (20, 22) as well as an electrically insulating surface section (24) separating the electrically conductive surface sections (20, 22) from each other,
- wherein the first electrically conductive surface section (20) of each contact means (16) electrically contacts the base body (10, 12) in an implanted state while the second electrically conductive surface section (22) of each contact means (16) is electrically insulated with respect to the base body (10, 12) by means of the electrically insulating surface section (24) in an implanted state,
- wherein each contact means (16) contains a coil arrangement (26) in its interior by means of which the electrically conductive surface sections (20, 22) of each contact means are electrically coupled, and
- wherein the coil arrangements (26) of the contact means (16) of a pair are wound in opposite directions.

2. The implantable device according to claim 1, **characterised in that** at least one contact means (16) of a pair is a fixation screw contacting the base body (10) with a screw head (28) and is screwed into the bone (18) with a screw shaft (30) in an implanted state.

3. The implantable device according to claim 1 or 2, **characterised in that** both contact means (16) of a pair are fixation screws.

4. The implantable device according to claim 2 or 3, **characterised in that** the electrically insulating surface section of the fixation screw (16) electrically insulates the screw head (28) with respect to the main part of the screw shaft (30).

5. The implantable device according to one of the preceding claims, **characterised in that** a plurality of pairs of shaft-shaped contact means (16) are provided, wherein respectively adjacent contact means (16) have coil arrangements (26) wound in opposite directions.

6. The implantable device according to one of the preceding claims, **characterised in that** the base body is an osteosynthesis means (10).

7. The implantable device according to claim 6, **characterised in that** the osteosynthesis means is an implantable bone support plate (10).

8. The implantable device according to one of the claims 1 to 5, **characterised in that** the base body is an endoprosthesis (12).

9. The implantable device according to claim 8, **characterised in that** the base body is the socket (12) of a joint replacement.

10. A system for generating localised electromagnetic fields in the area of an implant comprising
- an implantable device to be fixed to a bone, particularly according to one of the preceding claims, comprising
- an electrically conductive base body (10, 12) provided with at least two through holes (14) and
- at least one pair of shaft shaped contact means (16), wherein each contact means (16) passes through a through hole (14) of the base body (10, 12) and penetrates a section of the bone (18) in an implanted state,
- wherein the surface of each contact means (16) comprises a first and a second electrically conductive surface section (20, 22) as well as an electrically insulating surface section (24) separating the electrically conductive surface sections (20, 22) from each other,
- wherein the first electrically conductive surface section (20) of each contact means (16) electrically contacts the base body (10, 12) in an implanted state while the second electrically conductive surface section (22) of each contact means (16) is electrically insulated with respect to the base body (10, 12) by means of the electrically insulating surface section (24) in an implanted state,
- wherein each contact means (16) comprises a secondary coil arrangement (26) in its interior by means of which the electrically conductive surface sections (20, 22) of each contact means are electrically coupled, and
- wherein the secondary coil arrangements (26) of the contact means (16) of a pair are wound in opposite directions, and comprising
- a primary coil arrangement (32, 34, 36) adapted for generating an electromagnetic field in the area of the contact means (16) as well as
- an oscillation generator (62) for generating an alternating current in the primary coil arrangement (32, 34, 36).

11. The system according to claim 10, **characterised in that** the primary coil arrangement (36) includes a coil winding having a point of intersection (38) and defining two surfaces (40, 42) by means of an eight-shaped form, said surfaces (40, 42) being oriented with respect to each other at least during the application of the primary coil arrangement (36) so that the magnetic fields (44, 46) generated by a current flow in the primary coil arrangement (36) and passing through the surfaces are substantially unidirectional.

12. The system according to claim 11, **characterised in that** the primary coil arrangement (36) is elastically deformable so that the surfaces can be arranged on opposing sides of the implantable device.

13. The system according to claim 11 or 12, **characterised in that** fixation means (48, 50) cooperating at two positions of the primary coil arrangement opposed to the intersection point (38) are provided by means of which fixation means the orientation of the surfaces (40, 42) with respect to each other can be established and maintained.

14. The system according to claim 12 or 13, **characterised by** a coupling arrangement (60) by means of which the intersection point (38) of the primary coil arrangement is fixable.

15. The system according to one of the claims 12 to 14, **characterised in that** the position of the intersection point (38) and therefore the dimensions of the surfaces (40, 42) are variable.

## Revendications

1. Dispositif implantable pour être fixé à un os et comportant
- un corps de base conducteur d'électricité (10, 12) comportant au moins deux orifices de passage (14) et
- au moins une paire de moyens de contact (16) en forme de tige, chaque moyen de contact traversant une orifice de passage (14) du corps de base (10, 12) et pénétrant une zone de l'os (18) dans un état implanté,
- la surface de chaque moyen de contact (16) comportant une première et une deuxième partie de surface conductrice d'électricité (20, 22) ainsi qu'une partie de surface isolante d'électricité (24) qui sépare les parties de surface conductrices d'électricité (20, 22) l'une de l'autre,
- la première partie de surface conductrice d'électricité (20) de chaque moyen de contact (16) contactant électriquement le corps de base (10, 12) dans un état implanté tandis que la deuxième partie de surface conductrice d'électricité (22) de chaque moyen de contact (16) est isolé électriquement contre le corps de base (10, 12) par le biais de la partie de surface isolante d'électricité (24) dans un état implanté.
- chaque moyen de contact (16) contenant dans son intérieur un agencement à bobine (26) par le biais duquel les parties de surface (20, 22) conductrices d'électricité de chaque moyen de contact sont couplées électriquement, et
- les agencements à bobine (26) des moyens de contact (16) d'une paire étant bobinés dans des sens contraires.

2. Dispositif implantable selon la revendication 1, **caractérisé en ce qu'**au moins un moyen de contact (16) d'une paire est une vis de fixation qui contacte le corps de base (10) avec une tête de vis (28) et qui peut être vissée dans l'os avec une tige de vis (30).

3. Dispositif implantable selon la revendication 1 ou 2, **caractérisé en ce que** les deux moyens de contact (16) d'une paire sont des vis de fixation.

4. Dispositif implantable selon la revendication 2 ou 3, **caractérisé en ce que** la partie de surface isolante d'électricité de la vis de fixation (16) isole électriquement la tête de vis (28) contre la partie principale de la tige de vis (30).

5. Dispositif implantable selon l'une des revendications précédentes, **caractérisé en ce que** plusieurs paires de moyens de contact (16) en forme de tige sont prévues, des moyens de contact (16) respectivement avoisinants comportant des agencements à bobine (26) bobinés dans des sens contraires.

6. Dispositif implantable selon l'une des revendications précédentes, **caractérisé en ce que** le corps de base est un moyen d'ostéosynthèse.

7. Dispositif implantable selon la revendication 6, **caractérisé en ce que** le moyen d'ostéosynthèse est une plaque de soutien d'os implantable (10).

8. Dispositif implantable selon l'une des revendications 1 bis 5, **caractérisé en ce que** le corps de base est une endoprothèse (12).

9. Dispositif implantable selon la revendication 8, **caractérisé en ce que** le corps de base est la glène (12) d'une articulation artificielle.

10. Système pour générer des champs électromagnétiques localisés dans la zone d'un implant, comportant
- un dispositif implantable pour être fixé à un os, notamment selon l'une des revendications précédentes, comportant
- un corps de base conducteur d'électricité (10, 12) comportant au moins deux orifices de passage (14) et
- au moins une paire de moyens de contact (16) en forme de tige, chaque moyen de contact traversant une orifice de passage (14) du corps de base (10, 12) et pénétrant dans une zone de l'os (18) dans un état implanté,
- la surface de chaque moyen de contact (16) comportant une première et une deuxième partie de surface conductrice d'électricité (20, 22) ainsi qu'une partie de surface isolante d'électricité (24) qui sépare les parties de surface conductrices d'électricité (20, 22) l'une de l'autre,
- la première partie de surface conductrice d'électricité (20) de chaque moyen de contact (16) contactant électriquement le corps de base (10, 12) dans un état implanté tandis que la deuxième partie de surface conductrice d'électricité (22) de chaque moyen de contact (16) est isolé électriquement contre le corps de base (10, 12) par le biais de la partie de surface isolante électrique (24) dans un état implanté,
- chaque moyen de contact (16) contenant dans son intérieur un agencement à bobine secondaire (26) par le biais duquel les parties de surface conductrices d'électricité (20, 22) de chaque moyen de contact sont couplées électriquement, et
- les agencements à bobine secondaire (26) des moyens de contact (16) d'une paire étant bobinés dans des sens contraires et comportant
- un agencement à bobine primaire (32, 34, 36) pour générer un champ électromagnétique dans la zone des moyens de contact (16), ainsi qu'un
- générateur oscillateur (62) pour générer un courant alternatif dans l'agencement à bobine primaire (32, 34, 36).

11. Système selon la revendication 10, **caractérisé en ce que** l'agencement à bobine primaire (36) comporte un bobinage ayant un point de croisement (38) et définissant, par une forme de huit, deux surfaces (40, 42), les surfaces (40, 42) étant orientées mutuellement au moins pendant l'application de l'agencement à bobine primaire (36) de façon que les champs magnétiques (44, 46) qui sont générés par un flux de courant dans l'agencement à bobine primaire (36) et qui traversent les surfaces sont orientés essentiellement pareil.

12. Système selon la revendication 11, **caractérisé en ce que** l'agencement à bobine primaire (36) est déformable élastiquement de façon que les surfaces peuvent être agencées sur des côtés opposés du dispositif implantable.

13. Système selon la revendication 11 ou 12, **caractérisé en ce que** des moyens de fixation (48, 50) coopérants sont prévus dans deux positions de l'agencement à bobine primaire détournées du point de croisement (38) par le biais desquels l'orientation mutuelle des surfaces (40, 42) peut être établie et maintenue.

14. Système selon la revendication 12 ou 13, **caractérisé par** un dispositif de couplage (60) par le biais duquel le point de croisement (38) de l'agencement à bobine primaire peut être fixé.

15. Système selon l'une des revendications 12 à 14, **caractérisé en ce que** la position du point de croisement (38) et par conséquent les dimensions des surfaces (40, 42) sont variables.
